# EUROPEAN PATENT APPLICATION

(11) **EP 2 279 734 A1**
(43) Date of publication of application: **02.02.2011**
(21) Application number: 09750604.2
(22) Date of filing: 20.05.2009
(51) Int. Cl.: A61K 31/232, A61K 45/00, A61P 3/06, A61P 7/02, A61P 9/00, A61P 9/10

(54) **COMPOSITION FOR PREVENTING CARDIOVASCULAR EVENT IN HIGH-RISK PATIENT**

(30) Priority: 20.05.2008 JP 2008132448
(71) Applicant: MOCHIDA PHARMACEUTICAL CO., LTD., Shinjuku-ku Tokyo 160-8515 (JP)
(72) Inventor: YOKOYAMA, Mitsuhiro, Kobe-shi Hyogo 651-1112 (JP); ORIGASA, Hideki, Toyama-shi Toyama 930-0044 (JP); MATSUZAKI, Masunori, Ube-shi Yamaguchi 755-0152 (JP); MATSUZAWA, Yuji, Takarazuka-shi Hyogo 665-0804 (JP); SAITO, Yasushi, Chiba-shi Chiba 260-0853 (JP); ITAKURA, Hiroshige, Tokyo 108-0074 (JP)
(74) Representative: Grünecker, Kinkeldey, Stockmair & Schwanhäusser Anwaltssozietät
(86) International application number: PCT/JP2009/059277
(87) International publication number: WO 2009/142242

(57) **Abstract**

Provided is a pharmaceutical composition for reducing the risk in a patient at high risk for occurrence and/or recurrence of a cardiovascular event by administering the composition to the patient with a high ratio of oleic acid to stearic acid in plasma. The pharmaceutical composition contains ethyl icosapentate as its effective component. The composition is expected to reduce the risk in the patient at high risk for occurrence and/or recurrence of a cardiovascular event, and particularly to have a preventive effect on occurrence and/or recurrence of a cardiovascular event in the patient with a high ratio of oleic acid to stearic acid in plasma.

## Description

### TECHNICAL FIELD

This invention relates to a pharmaceutical composition for reducing the risk in patients at high risk for occurrence and/or recurrence of cardiovascular events, the pharmaceutical composition containing at least ethyl icosapentate (hereinafter abbreviated as EPA-E). The invention also relates to a method for selecting a high-risk patient in which the ratio of oleic acid to stearic acid in plasma is used as a marker.

### BACKGROUND ART

Westernization of diet has resulted in an increase of patients suffering from lifestyle-related diseases such as diabetes, hyperlipidemia, and hypertension. Some of these diseases finally lead to arteriosclerotic diseases such as myocardial infarction, angina pectoris, and cerebral infarction. Treatment of the lifestyle-related diseases is based on the improvement of lifestyle, and more specifically, on the alimentary therapy and kinesitherapy. However, such improvement of the dietary life or the lack of exercise is often difficult in the patients suffering from the lifestyle-related diseases, and they usually start pharmacotherapy in order to prevent poor prognosis, for example, occurrence of myocardial infarction or cerebral infarction.

Polyunsaturated fatty acids are known as exemplary compounds having an improving effect. Fatty acids are classified into saturated fatty acids and unsaturated fatty acids, and unsaturated fatty acids are further classified into monounsaturated fatty acids and polyunsaturated fatty acids. Exemplary saturated fatty acids include myristic acid (14:0), palmitic acid (16:0) and stearic acid (18:0, hereinafter abbreviated as SA) and animal fat contains a large amount of SA. An exemplary monounsaturated fatty acid includes oleic acid (18:1, hereinafter abbreviated as OA). Olive oil contains a large amount of OA. When ingested, OA allegedly has the action of lowering the so-called bad cholesterol levels (LDL) without lowering the so-called good cholesterol levels (HDL). The polyunsaturated fatty acids are defined as fatty acids having two or more carbon-carbon double bonds in the molecule, and the polyunsaturated fatty acids are further grouped by the positions of the double bonds into ω-3, ω-6, and other polyunsaturated fatty acids. Examples of the ω-3 polyunsaturated fatty acids include α-linolenic acid (18:3), icosapentaenoic acid (20:5, hereinafter abbreviated as EPA), and docosahexaenoic acid (22:6, hereinafter abbreviated as DHA), and examples of the ω-6 polyunsaturated fatty acids include linoleic acid (18:2), γ-linolenic acid (18:3), and arachidonic acid (20:4, hereinafter referred to as AA). For example, fish oil contains a large amount of EPA.

These fatty acids are known to be components of cell membranes and also to be materials of various physiologically active components in a living body. Some fatty acids have physiological activity. For example, EPA is known to exhibit various actions including antiarteriosclerotic action, platelet aggregation inhibitory action, hypolipidemic action, antiinflammatory action, antitumor action, and central action and is also available as drugs. On the other hand, fatty acids that may lead to arteriosclerosis by excess intake are also known to exist. Humans cannot synthesize polyunsaturated fatty acids in the body and therefore require dietary intake of such polyunsaturated fatty acids.

The fatty acids taken in from the diet are mainly absorbed from the intestinal tract and mainly exist in chylomicron in blood. Triglycerides synthesized in the liver bind to proteins and are released into blood as very low density lipoproteins (VLDL). The plasma fatty acid composition can be determined by clinical test. This test is covered by insurance (test execution: 450 points; analysis of the biochemical test (II): 144 points).

Many reports have been made on the merits and demerits of the fatty acids taken in from the diet, but few reports discuss the relation between the plasma fatty acid concentration or the ratio between specific fatty acids and the risk for diseases.

There is a report on the plasma fatty acid composition and coronary artery diseases based on the results of the subgroup analysis of a large scale clinical trial (JELIS, see Non-Patent Literature 1) using high-purity EPA-E. For example, it is described that the higher the plasma EPA concentration is, the more the risk for coronary artery diseases are reduced (see Non-Patent Literature 2). On the other hand, there is a literature which determines the fatty acid composition in the serum of 42 healthy subjects and discusses the correlation with the coronary artery risk factors (e.g., BMI, blood pressure and blood glucose level). This literature describes that an increased serum nervonic acid (24:1) reduces the risk, whereas an increased serum EPA concentration increases the risk (see Non-Patent Literature 3). As described above, the relation between the plasma fatty acids and the risk for diseases is not elucidated yet, but these literatures do not disclose or suggest the relation between the OA/SA ratio and the risk for coronary artery diseases, and the use of the OA/SA ratio as a marker for the coronary artery diseases.

### CITATION LIST

### NON PATENT LITERATURE

Non-Patent Literature 1: Lancet, vol. 369, pp. 1090-1098 (2007)
Non-Patent Literature 2: JACC, vol. 49, No. 9, Suppl. A, p. 344 (2007)
Non-Patent Literature 3: Int Heart J., vol., 46, No. 6, pp. 975-985 (2005)

### SUMMARY OF INVENTION

### TECHNICAL PROBLEMS

An object of the invention is to provide a method for selecting a high-risk patient who may easily develop a cardiovascular event, a method for managing such a high-risk patient, and a pharmaceutical composition for managing such a high-risk patient or a pharmaceutical composition for preventing occurrence and/or recurrence of a cardiovascular event in such a high-risk patient.

### SOLUTION TO PROBLEMS

The inventors of the invention made an intensive study to solve the above problems and found that specific fatty acids in plasma or their ratio serves as a disease risk marker. It was also found that the specific fatty acids in plasma or their ratio can be used as a criterion to manage patients at high risk for diseases or determine the drug's therapeutic effect. It was further found that the ratio of OA to SA in plasma is a particularly excellent marker for selecting a patient at high risk for occurrence and/or recurrence of cardiovascular events. In addition to a method for selecting a high-risk patient based on the OA/SA ratio and a method for managing a high-risk patient based on the OA/SA ratio, it was further found that the treatment method and the drug's effect can be determined based on the OA/SA ratio. It was further found that a pharmaceutical composition containing EPA or EPA-E as its effective component reduces the OA/SA ratio and completed the invention of a pharmaceutical composition for managing such high-risk patients or a pharmaceutical composition for preventing occurrence and/or recurrence of cardiovascular events in the high-risk patients. Accordingly, the invention provides:
(1) A pharmaceutical composition for reducing an OA/SA ratio in plasma of a patient, the pharmaceutical composition containing EPA-E as its effective component.
   More specifically, the invention provides the following:
(2) The pharmaceutical composition according to (1), wherein the OA/SA ratio in plasma of the patient is high.
(3) The pharmaceutical composition according to (1) or (2) for reducing the risk of the patient at high risk for occurrence and/or recurrence of cardiovascular events.
(4) The pharmaceutical composition according to any one of (1) to (3), wherein the patient suffers from hyperlipidemia and/or dyslipidemia.
(5) The pharmaceutical composition according to (1) or (4) for administering to the patient with an OA/SA ratio in plasma of at least 3.
(6) The pharmaceutical composition according to any one of (1) to (5), wherein administration of the composition to the patient with an OA/SA ratio in plasma of at least 3 is started, continued until the OA/SA ratio in plasma is reduced to less than 2.6 and further continued so that the OA/SA ratio in plasma is kept at less than 2.6.
(7) The pharmaceutical composition according to any one of (1) to (6), wherein fatty acid fractions in total lipids are measured to specify the patient with a high OA/SA ratio in plasma.
(8) The pharmaceutical composition according to any one of (1) to (7) which is used in combination with a 3-hydroxy-3-methylglutaryl-coenzyme A reductase inhibitor (hereinafter abbreviated as "HMG-CoA RI").
(9) A high-risk patient selecting method for selecting a patient at high risk for occurrence and/or recurrence of cardiovascular events, wherein an OA/SA ratio in plasma is used as a marker.
(10) A method for preventing occurrence and/or recurrence of cardiovascular events by keeping at less than 2.6 an OA/SA ratio in plasma of a patient who has a high OA/SA ratio in plasma.
(11) The method according to (10) which comprises administering a pharmaceutical composition containing EPA-E as its effective component.
(12) A method for preventing occurrence and/or recurrence of cardiovascular events in humans, wherein EPA-E is administered to keep a ratio of oleic acid to stearic acid in human plasma at less than 2.6.
(13) A method for preventing occurrence and/or recurrence of cardiovascular events, the method comprising the steps of:
   1) collecting blood from patients;
   2) measuring oleic acid and stearic acid levels in plasma;
   3) calculating a ratio of oleic acid to stearic acid (OA/SA ratio);
   4) selecting patients with a ratio of oleic acid to stearic acid of at least 3;
   5) administering a pharmaceutical composition containing EPA-E as its effective component to the selected patients;
   6) periodically measuring the ratio of oleic acid to stearic acid in plasma during administration of the pharmaceutical composition; and
   7) continuing the administration of EPA-E until the ratio of oleic acid to stearic acid is reduced to less than 2.8.
(14) The method according to (13), wherein the pharmaceutical composition containing EPA-E as its effective component is used in combination with HMG-CoA RI.
(15) The method according to (13) or (14), wherein the administration is continued until the ratio of oleic acid to stearic acid is reduced to less than 2.6.
(16) The method according to (13) to (15), wherein prevention is to reduce the occurrence rate and/or recurrence rate of the cardiovascular events.

### ADVANTAGEOUS EFFECTS OF INVENTION

Patients at high risk for occurrence of specific diseases and particularly patients at high risk for occurrence of cardiovascular events can be selected based on the plasma fatty acids or their ratio. In particular, the OA/SA ratio is useful as a marker for selecting such high-risk patients and the OA/SA ratio can be used as a criterion to manage the high-risk patients and determine the therapeutic effects. In addition, the pharmaceutical composition of the invention containing EPA or EPA-E is useful as a pharmaceutical composition for reducing the OA/SA ratio in plasma and is also useful as a drug for reducing the risk for occurrence and/or recurrence of cardiovascular events in patients with a high OA/SA ratio.
By using the pharmaceutical composition of the invention in combination with HMG-CoA RI, its effects are synergistically enhanced. The pharmaceutical composition can be expected to further enhance the effect of preventing occurrence and/or recurrence of cardiovascular events and is of clinical benefit.

### DESCRIPTION OF EMBODIMENTS

Next, the present invention is described in detail.
A first aspect of the invention provides a pharmaceutical composition for reducing an OA/SA ratio in plasma of a patient, the pharmaceutical composition containing EPA-E as its effective component. More specifically, the pharmaceutical composition is administered to a patient who has a high OA/SA ratio in plasma and preferably an OA/SA ratio in plasma of at least 3. Alternatively, the pharmaceutical composition is administered to a patient who has an OA/SA ratio in plasma of at least 3.5. More specifically, the pharmaceutical composition is used to reduce the risk of the patient at high risk for occurrence and/or recurrence of a cardiovascular event, and preferably to prevent occurrence and/or recurrence of a cardiovascular event. Examples of the subject to which the composition is to be administered include all humans who require reduction of the OA/SA ratio in plasma, and particularly includes patients with hyperlipidemia and/or dyslipidemia and among others patients with hypercholesterolemia.

A second aspect of the invention provides a pharmaceutical composition of which administration to a patient with a high OA/SA ratio in plasma and preferably a patient with an OA/SA ratio in plasma of at least 3 is started, continued until the OA/SA ratio in plasma is reduced to less than 2.6 and further continued so that the OA/SA ratio in plasma is kept at less than 2.6. Alternatively, the invention provides a pharmaceutical composition of which administration to a patient with a high OA/SA ratio in plasma and preferably a patient with an OA/SA ratio in plasma of at least 3 is started, continued until the OA/SA ratio in plasma is reduced to less than 2.8 and further continued so that the OA/SA ratio in plasma is kept at less than 2.8. The pharmaceutical composition preferably contains at least EPA-E as its effective component. More preferably, administration of the pharmaceutical composition is continued until the OA/SA ratio in plasma is reduced to less than 2 and further continued so that the OA/SA ratio in plasma is kept at less than 2. Even more preferably, administration of the pharmaceutical composition is continued until the OA/SA ratio in plasma is reduced to less than 1.5 and further continued so that the OA/SA ratio in plasma is kept at less than 1.5. Still more preferably, administration of the pharmaceutical composition is continued until the OA/SA ratio in plasma is reduced to less than 1 and further continued so that the OA/SA ratio in plasma is kept at less than 1. Most preferably, administration of the pharmaceutical composition is continued until the OA/SA ratio in plasma is reduced to less than 0.5 and further continued so that the OA/SA ratio in plasma is kept at less than 0.5.

A third aspect of the invention provides a pharmaceutical composition for reducing an OA/SA ratio in plasma of a patient, the pharmaceutical composition containing at least EPA-E as its effective component and used in combination with HMG-CoA RI. The pharmaceutical composition is preferably administered to a patient with a high OA/SA ratio in plasma and more specifically a patient with an OA/SA ratio in plasma of at least 3. The pharmaceutical composition of the invention contains at least EPA-E and has the effect of preventing occurrence and/or recurrence of a cardiovascular event by administering to a human with a high OA/SA ratio in plasma, that is, a human at high risk for occurrence and/or recurrence of the cardiovascular event. In particular, the pharmaceutical composition has the effect of preventing occurrence and/or recurrence of a cardiovascular event in a patient with hypercholesterolemia having undergone treatment with HMG-CoA RI. The pharmaceutical composition of the invention has the combination effect with HMG-CoA RI and can further prevent occurrence and/or recurrence of a cardiovascular event by combination use.

A fourth aspect of the invention provides a high-risk patient selecting method for selecting a patient at high risk of occurrence and/or recurrence of a cardiovascular event, for example, a patient with a high OA/SA ratio or a patient with an increased OA/SA ratio in plasma than in the previous measurement. In this method, the OA/SA ratio in plasma is used as a marker. More specifically, blood is collected from patients; and the OA and SA levels are measured by a technique such as fractionation of fatty acids into 24 fractions to calculate the OA/SA ratio in plasma, which is then used as a marker.
Another embodiment of the fourth aspect of the invention provides a high-risk patient selecting method for selecting a patient at high risk for occurrence and/or recurrence of a cardiovascular event, the fatty acid in each of 24 plasma fatty acid fractions being used as a marker. Specific examples of the marker include myristic acid, palmitic acid, palmitoleic acid (16:1), stearic acid and oleic acid. Oleic acid is preferred.
Still another embodiment of the fourth aspect of the invention provides a high-risk patient selecting method for selecting a patient at high risk for occurrence and/or recurrence of a cardiovascular event, the ratio of the respective fatty acids in the 24 plasma fatty acid fractions being used as a marker. Specific examples of the ratio of the fatty acids include a ratio of oleic acid to stearic acid, a ratio of palmitoleic acid to palmitic acid, a ratio of stearic acid to myristic acid, a ratio of palmitic acid to myristic acid, and a ratio of stearic acid to palmitic acid. The ratio of oleic acid to stearic acid is preferred.
Patients with a high OA/SA ratio in plasma and preferably patients with an OA/SA ratio in plasma of at least 3 have a high risk for occurrence and/or recurrence of cardiovascular events and require a special management. First of all, patients with underlying diseases such as hyperlipidemia and dyslipidemia receive dietary therapy and exercise therapy, but patient selection based on the criterion that the OA/SA ratio in plasma is at least 3 enables a high-risk patient to be selected to start a proper drug therapy at an earlier stage. Patients with an OA/SA ratio in plasma of at least 3.5 have a higher risk. Occurrence of cardiovascular events can be suppressed by selecting such patients at an earlier stage and managing the patients by combination of a plurality of drugs.

A fifth aspect of the invention provides a method for preventing occurrence and/or recurrence of a cardiovascular event by keeping at less than 2.6 the OA/SA ratio in plasma of a patient who has a high OA/SA ratio in plasma. More specifically, blood is collected from a patient periodically and preferably every month; and the OA and SA levels are measured by a technique such as fractionation of fatty acids into 24 fractions to calculate the OA/SA ratio in plasma. When the OA/SA ratio exceeds 2.6, the patient is treated with lifestyle guidance and medication so that the OA/SA ratio is reduced to less than 2.6. Alternatively, there is provided a method for preventing occurrence and/or recurrence of a cardiovascular event by keeping at less than 2.8 the OA/SA ratio in plasma of a patient who has a high OA/SA ratio in plasma. More specifically, blood is collected from a patient periodically and preferably every month and the OA and SA levels are measured by a technique such as fractionation of fatty acids into 24 fractions to calculate the OA/SA ratio in plasma. When the OA/SA ratio exceeds 2.8, the patient is treated with lifestyle guidance and medication so that the OA/SA ratio is reduced to less than 2.8. The treatment is not particularly limited but a method with which a pharmaceutical composition containing EPA-E as its effective component is administered is preferred. A method with which the OA/SA ratio in plasma is kept at less than 2 is more preferred, and a method with which the OA/SA ratio in plasma is kept at less than 1.5 is even more preferred.
Another embodiment of the fifth aspect of the invention provides a method for preventing occurrence and/or recurrence of a cardiovascular event by keeping the OA/SA ratio in plasma of a patient with a high OA/SA ratio in plasma at such a level as to prevent occurrence and/or recurrence of a cardiovascular event. More specifically, according to the method, the OA/SA ratio in plasma is kept at less than 2, more preferably less than 1.5, even more preferably less than 1 and most preferably less than 0.5. The test results of the invention confirmed that occurrence of a cardiovascular event is prevented by adjusting the OA/SA ratio in plasma to less than 1.8. There is no particular limitation on the specific method for keeping the OA/SA ratio in plasma of a patient with a high OA/SA ratio in plasma at such a value as to prevent occurrence and/or recurrence of a cardiovascular event, but an exemplary method involves administering a pharmaceutical composition containing EPA-E as its effective component to the patient.

A sixth aspect of the invention provides a method for preventing occurrence and/or recurrence of a cardiovascular event which comprises administering a composition containing at least EPA-E as its effective component to a patient with a high OA/SA ratio in plasma for at least two consecutive years. According to the preventive method, the composition is preferably administered for at least three consecutive years and more preferably for at least five consecutive years.

A seventh aspect of the invention provides a pharmaceutical composition containing ethyl icosapentate as its effective component, the pharmaceutical composition being administered to a patient with a higher OA/SA ratio in plasma than in the previous measurement and preferably a patient with a higher OA/SA ratio in plasma by at least 0.5 than in the previous measurement to prevent occurrence and/or recurrence of a cardiovascular event. More specifically, the pharmaceutical composition is administered to a patient with a higher OA/SA ratio in plasma more preferably by at least 1.0 and even more preferably by at least 1.5 than in the previous measurement.
Another embodiment of the seventh aspect of the invention provides a pharmaceutical composition which is continuously administered to a patient with a higher OA/SA ratio in plasma than in the previous measurement until the OA/SA ratio in plasma is reduced to a value lower than that of the previous measurement and further continuously administered so as to keep the OA/SA ratio in plasma below the value of the previous measurement.

An eight aspect of the invention provides a pharmaceutical composition containing ethyl icosapentate as its effective component, the pharmaceutical composition being administered to a patient with a high plasma oleic acid level or plasma stearic acid level to prevent occurrence and/or recurrence of a cardiovascular event. The pharmaceutical composition is more preferably administered to a patient with a plasma OA level of at least 22 mol% or a plasma SA level of at least 6.9 mol%, even more preferably to a patient with a plasma OA level of at least 22 mol% or a plasma SA level of at least 7.4 mol%.
The pharmaceutical composition is preferably administered for at least two consecutive years, more preferably at least three consecutive years and even more preferably at least five consecutive years.

A ninth aspect of the invention provides a method for preventing occurrence and/or recurrence of a cardiovascular event which involves collecting blood from patients, measuring OA and SA levels by a technique such as fractionation of fatty acids into 24 fractions to calculate the OA/SA ratio in plasma of the patients, selecting patients with a high OA/SA ratio in plasma, and administering a pharmaceutical composition containing at least EPA-E as its effective component to the selected patients.
Another embodiment of the ninth aspect of the invention provides a method for reducing the risk in patients at high risk for occurrence and/or recurrence of cardiovascular events which involves collecting blood from patients, measuring OA and SA levels by a technique such as fractionation of fatty acids into 24 fractions to calculate the OA/SA ratio in plasma of the patients, selecting patients with a high OA/SA ratio in plasma, and administering a pharmaceutical composition containing at least EPA-E as its effective component to the selected patients.

The terms used in the description of the respective aspects of the invention are described below in further detail.

The ratio of oleic acid (OA) to stearic acid (SA) in plasma can be calculated by collecting blood from a patient and measuring the levels of the individual fatty acids such as OA and SA or the ratio of the individual fatty acids in the total fatty acids by, for example, gas chromatography. The OA/SA ratio can be also calculated from the individual fatty acid levels obtained by performing a clinical test item, that is, fractionation of fatty acids in total lipids which can be performed by, for example, SRL, Inc. The measurement method and calculation method are not particularly limited.
The fatty acid fractionation into 24 fractions is a testing method which involves fractionating fatty acids including unsaturated fatty acids into specific 24 fractions and quantitatively determining the fractions by gas chromatography. More specifically, the plasma fatty acids are extracted by the method of, for example, Folch et al. (Folch J. et al., J. Biol. Chem. 226, 497-509 (1957)). It is possible to use tricosanoic acid (C23:0) as an internal standard, to convert each fatty acid into a methyl ester by addition of boron trifluoride and methanol, and to quantitatively determine the methyl ester of each fatty acid by measurement using a gas chromatograph such as SHIMAZU GC-17A (Shimadzu Corporation) and a capillary column such as BPX70 (with an internal diameter of 0.25 mm and a length of 30 m; SGE International Ltd.). However, this is not the sole method of the invention.

The patient with a high OA/SA ratio in plasma refers to a human patient with an OA/SA ratio in plasma of at least 3, more preferably at least 3.5, even more preferably at least 4 and most preferably at least 5 irrespective of whether or not there is a physical symptom.
Examples of the cardiovascular event include cardiovascular death (fatal myocardial infarction, sudden cardiac death, etc.), nonfatal myocardial infarction, percutaneous transluminal coronary angioplasty (PTCA), percutaneous coronary intervention (PCI), AC bypass grafting and other cardiovascular revascularization, new occurrence of rest angina and exercise-induced angina, and destabilization of angina (caused by hospitalization, PTCA, PCI, AC bypass grafting and other cardiovascular revascularization).

The expression "preventing occurrence and/or recurrence of cardiovascular events" refers to preventing occurrence of cardiovascular events in humans having no history of the cardiovascular events (primary prevention) and/or preventing recurrence of cardiovascular events in humans having a history of the cardiovascular events (secondary prevention).

In addition to the prevention of occurrence and/or recurrence of cardiovascular events, for example, the prophylactic and therapeutic effect of arteriosclerotic diseases, the effect of improving metabolic syndrome, the prophylactic and therapeutic effect of neuropsychiatric diseases, the prophylactic and therapeutic effect of various age-related diseases and the prophylactic and therapeutic effect of metabolic diseases are expected by reducing the OA/SA ratio in plasma.

The term "icosapentaenoic acid" designates all-cis-5,8,11,14,17-icosapentaenoic acid. Ethyl icosapentate is ethyl ester of the icosapentaenoic acid.
EPA or its salt may be used instead of EPA-E as the effective component of the pharmaceutical composition of the invention.

The content ratio of EPA-E to the total fatty acids in the pharmaceutical composition of the invention and the dosage of EPA-E are not particularly limited as long as the intended effects of the invention are attained. However, EPA-E preferably has a high purity. For example, the content ratio of EPA-E to the total fatty acids and derivatives thereof is preferably at least 40 wt%, more preferably at least 90 wt% and even more preferably at least 96.5 wt%. Epadel^{™} and Epadel S^{™} commercially available in Japan from Mochida Pharmaceutical Co., Ltd. as therapeutic agents for arteriosclerosis obliterans and hyperlipidemia are soft capsules containing high purity EPA-E at a content ratio of EPA-E to the total fatty acids of at least 96.5 wt%. These may be used for the pharmaceutical composition of the invention. The daily dose of the inventive pharmaceutical composition in terms of EPA-E is typically 0.3 to 6 g/day, preferably 0.9 to 3.6 g/day, and even more preferably 1.8 to 2.7 g/day. Other preferable daily doses are 0.3 to 2.7 g/day and 0.3 to 1.8 g/day.

Another preferred example of the fatty acid that may be contained in the pharmaceutical composition of the invention includes ethyl docosahexaenoate (hereinafter referred to as "DHA-E"). While the compositional ratio of EPA-E/DHA-E, the content ratio of EPA-E and DHA-E (hereinafter referred to as "EPA-E + DHA-E") to the total fatty acids, and dosage of EPA-E + DHA-E are not particularly limited as long as the intended effects of the invention are attained, the composition is preferably the one having a high purity of EPA-E and DHA-E, for example, the one having a content ratio of EPA-E + DHA-E to the total fatty acids and derivatives thereof of preferably at least 40 wt%, more preferably at least 80 wt%, and even more preferably at least 90 wt%. The daily dose in terms of EPA-E + DHA-E is typically 0.3 to 10 g/day, preferably 0.5 to 6 g/day, and more preferably 1 to 4 g/day. Other preferable daily doses are 0.3 to 6 g/day, 0.3 to 4 g/day and 0.3 to 1 g/day. The content of other long chain saturated fatty acids is preferably low, and among the long chain unsaturated fatty acids, the content of ω-6 fatty acids, and in particular, the content of arachidonic acid is preferably as low as less than 2 wt%, and more preferably less than 1 wt%. The soft capsule (Lovaza^{™} available from Reliant and Pronova) containing about 46 wt% of EPA-E and about 38 wt% of DHA-E is commercially available in the U.S., Europe, and other countries as a commercial drug containing EPA-E and DHA-E for treating hypertriglyceridemia. This may be used for the pharmaceutical composition of the invention.

Since EPA-E and DHA-E are both highly unsaturated, the pharmaceutical composition of the invention desirably includes an effective amount of an antioxidant such as butylated hydroxytoluene, butylated hydroxyanisole, propyl gallate, gallic acid, pharmaceutically acceptable quinone, or α-tocopherol.

The term "hyperlipidemia patient" means a patient with an increased serum T-Cho concentration or serum LDL-Cho concentration, a decreased serum HDL-Cho concentration, or an increased serum TG. In a narrower sense, this term means a patient satisfying one of hypercholesterolemia (with a serum T-Cho concentration of at least about 220 mg/dL, and in more strict sense, at least 250 mg/dL), high LDL cholesterolemia (with a serum LDL-Cho concentration of at least 140 mg/dL), low HDL-cholesterolemia (with a serum HDL-Cho concentration of less than 40 mg/dL) and hypertriglyceridemia (with a serum TG concentration of at least 150 mg/dL). The serum lipid concentrations may be usually measured and calculated by a known method using a blood sample collected during fasting. An example of the subject to which the pharmaceutical composition of the invention is preferably administered include a hypercholesterolemia patient. The term "hypercholesterolemia patient" as used herein means a patient with an increased serum T-Cho concentration or serum LDL-Cho concentration. In a narrower sense, this term means a patient suffering from hypercholesterolemia (with a serum T-Cho concentration of at least about 220 mg/dL, and in more strict sense, at least 250 mg/dL) or high LDL cholesterolemia (with a serum LDL-Cho concentration of at least 140 mg/dL).

The term "dyslipidemia" refers to a condition which satisfies at least one of high LDL cholesterolemia (i.e. fasting serum LDL cholesterol value of at least 140 mg/dL), low HDL cholesterolemia (i.e. fasting serum HDL cholesterol value of less than 40 mg/dL), and hypertriglyceridemia (i.e. fasting serum triglyceride value of at least 150 mg/dL) according to the diagnostic criteria described in "Guideline for Preventing Arteriosclerotic Diseases, 2007" (edited and published by Japan Atherosclerosis Society).

While HMG-CoA RI includes all those having inhibitory action for 3-hydroxy-3-methylglutaryl coenzyme A reductase, it is preferred to use a pharmaceutically administrable inhibitor. More specifically, the HMG-CoA RI is preferably at least one member selected from the group consisting of pravastatin, simvastatin, lovastatin, fluvastatin, cerivastatin, atorvastatin, pitavastatin, rosuvastatin, and salts and derivatives thereof, and is more preferably pravastatin, lovastatin, simvastatin, fluvastatin, atorvastatin, pitavastatin, or rosuvastatin, and is even more preferably pravastatin or simvastatin. All salts are included as long as they are pharmaceutically administrable, and sodium and calcium salts such as pravastatin sodium, fluvastatin sodium, cerivastatin sodium, atorvastatin calcium, pitavastatin calcium, and rosuvastatin calcium are particularly preferred. In the practice of the invention, "pravastatin", for example, also includes the pravastatin in the form of a salt unless otherwise noted.

The HMG-CoA RI is preferably used according to the dosage regimen recommended for the particular drug used, and the dose may be adequately increased or decreased depending on the type, dosage form, administration route, daily frequency, severity of the symptoms, body weight, gender, age, and the like. When orally administered, the HMG-CoA RI is administered at a dose of 0.05 to 200 mg/day, and preferably 0.1 to 100 mg/day in a single dose or in two divided doses. If necessary, the total dose may be administered in several divided doses. The dose of the HMG-CoA RI may be reduced depending on the dose of the EPA-E.

It is to be noted that pravastatin sodium (Mevalotin^{™} tablets and fine granules, Daiichi Sankyo Co., Ltd.), simvastatin (Lipovas^{™} tablets, Banyu Pharmaceutical Co., Ltd.), fluvastatin sodium (Lochol^{™} Tablets, Novartis Pharma K.K.), atorvastatin calcium hydrate (Lipitor^{™} tablets, Astellas Pharma Inc. and Pfizer Inc.), pitavastatin calcium (Livalo^{™}, Kowa Pharmaceutical Co., Ltd. and Daiichi Sankyo Co., Ltd.), and rosuvastatin calcium (Crestor^{™} tablets, AstraZeneca KK and Shionogi & Co., Ltd.) are commercially available in Japan as drugs for treating hyperlipidemia, and lovastatin (Mevacor^{™} tablets, Merck) is commercially available in the U.S. as a drug for treating hyperlipidemia. These drugs may be purchased and used according to the prescribed dosage regimen.

The daily dose of pravastatin sodium is preferably 5 to 60 mg and more preferably 10 to 20 mg. The daily dose of simvastatin is preferably 2.5 to 60 mg and more preferably 5 to 20 mg. The daily dose of fluvastatin sodium is preferably 10 to 180 mg and more preferably 20 to 60 mg. The daily dose of atorvastatin calcium hydrate is preferably 5 to 120 mg and more preferably 10 to 40 mg. The daily dose of pitavastatin calcium is preferably 0.5 to 12 mg and more preferably 1 to 4 mg. The daily dose of rosuvastatin calcium is preferably 1.25 to 60 mg and more preferably 2.5 to 20 mg. The daily dose of lovastatin is preferably 5 to 160 mg and more preferably 10 to 80 mg. The daily dose of cerivastatin sodium is preferably 0.075 to 0.9 mg and more preferably 0.15 to 0.3 mg. However, the dose is not limited to those as described above.

The expression "combined use of EPA-E with HMG-CoA RI" as used herein include the embodiment in which EPA-E and HMG-CoA RI are simultaneously administered and the embodiment in which both agents are separately administered. When these agents are simultaneously administered, they may be formulated either as a single combined drug or separate drugs. When these agents are separately administered, EPA-E may be administered either before or after HMG-CoA RI. The doses and ratio of EPA-E and HMG-CoA RI may be adequately set.

The pharmaceutical composition and the preventive method of the present invention may be used with other drugs, for example, antiplatelet drugs such as aspirin, ticlopidine, clopidogrel, prasugrel, and cilostazol; anticoagulants such as warfarin, heparin, and ximelagatran; antihypertensive drugs such as angiotensin II receptor antagonists (candesartan, losartan, valsartan, etc.), angiotensin converting enzyme inhibitors, calcium channel antagonists (amlodipine, cilnidipine, etc.), and α1 blockers; diabetes drugs or abnormal glucose tolerance stimulants such as α-glucosidase inhibitors (voglibose, acarbose, etc.), biguanide drugs, thiazolidinedione drugs (pioglitazone, rosiglitazone, rivoglitazone, etc.), and prompt insulin release promoters (mitiglinide, nateglinide, etc.); antihyperlipidemic drugs and antiarteriosclerotic drugs such as HMG-CoA RI as described above, fibrate drugs, squalene synthetase inhibitors (TAK-475, etc.), and cholesterol absorption inhibitors (ezetimibe, etc.), probucol, anion exchange resin, nicotinic acid drugs, phytosterol, elastase, dextran sulfate sodium sulfur, pantothenic acid, and polyenephosphatidylcholine. Combinations with HMG-CoA RI or fibrate drugs are particularly useful.

In addition, EPA-E has not only the action of reducing the serum T-Cho concentration and the serum TG, but also the action of suppressing platelet aggregation based on inhibition of arachidonic acid cascade, the latter action being a pharmacological action different from the action HMG-CoA RI or fibrate drugs have. Therefore, the action as described above can also be exerted by combined administration with HMG-CoA RI or fibrate drugs.

The preparation may be orally administered to patients in the dosage form of tablet, capsule, microcapsule, granules, fine granules, powder, oral liquid preparation, syrup, or jelly. Preferably, the preparation is orally administered by filling in a capsule such as soft capsule or microcapsule. Most preferably, the preparation is encapsulated into substantially spherical, seamless soft capsules with a diameter of about 4 mm and orally administered.
The dosage and administration period of the pharmaceutical composition of the invention are determined so as to be sufficient for the expression of the intended action and may be adequately increased or decreased depending on the dosage form, administration route, daily frequency, severity of the symptoms, body weight, age, and the like. When orally administered, the pharmaceutical composition is administered at a dose in terms of EPA-E of 0.3 to 6 g/day, preferably 0.9 to 3.6 g/day, and more preferably 1.8 to 2.7 g/day. However, the composition may be administered if necessary in a single dose or in several divided doses although typically administered in three divided doses. In another embodiment of oral administration, the pharmaceutical composition may be administered at a dose in terms of EPA-E of 0.3 to 2.7 g/day and preferably 0.3 to 1.8 g/day. However, the composition may be administered if necessary in several divided doses although typically administered in a single dose. The pharmaceutical composition is preferably administered during or after the meal, and more preferably, immediately (within 30 minutes) after the meal. When such dose is orally administered, the administration period is typically at least 1 year, preferably at least 2 years, more preferably at least 3 years and even more preferably at least 5 years. The administration, however, is preferably continued as long as there is a considerable risk for occurrence and/or recurrence of the cardiovascular events. If necessary, drug holidays of about 1 day to about 3 months, and preferably about 1 week to about 1 month may be given.

### Examples

Next, the effects of the composition of the invention are demonstrated by referring to Examples, which by no means limit the scope of the present invention. (Example 1) Plasma OA/SA Ratio as Cardiovascular Event Risk, and Effect of EPA-E Administration on Plasma OA/SA Ratio

### Trial procedure

This trial corresponds to a partial analysis of the results obtained in JELIS (Japan EPA Lipid Intervention Study) which is a large scale clinical trial of high purity EPA preparation which was presented in the American College of Cardiology 2005 Annual Meeting (for general information on JELIS, see Lancet, vol. 369, pp. 1090-1098 (2007)). More specifically, of 18,645 subject patients of the JELIS trial (EPA group (9,326 cases) and control group (9,319 cases)), 15534 cases (sum of the control group and EPA group) were analyzed for the composition of plasma fatty acids and divided based on the average of the plasma OA/SA ratio during the whole observation period into three groups (i.e., a group having an OA/SA ratio of less than 2.6, a group having an OA/SA ratio of at least 2.6 but less than 3.0, and a group having an OA/SA ratio of at least 3.0) so that the three groups were approximately equal in number, and the groups were compared for the incidence of cardiovascular events for 5 years from the start of administration. The comparison results are shown in Table 1.
The "corrected hazard ratio" refers to a hazard ratio after correction of other factors that may affect the evaluation of the relation between the OA/SA ratio and the occurrence of cardiac events (dispersion due to, for example, gender difference and whether or not there is another underlying disease).

The EPA group was orally administered with Epadel (EPA-E available from Mochida Pharmaceutical Co., Ltd.) typically at an adult dose of 600 mg per administration and 3 times a day immediately after the meal. However, in the case of abnormal serum TG, the dose could be increased to 900 mg per administration and 3 times a day depending on the degree of abnormality. In both groups, pravastatin sodium (Mevalotin^{™} tablets and fine granules, Daiichi Sankyo Co., Ltd.), simvastatin (Lipovas^{™} tablets, Banyu Pharmaceutical Co., Ltd.), or atorvastatin calcium hydrate (Lipitor^{™} tablets, Astellas Pharma Inc. and Pfizer Inc.) was used for the base drug, and these drugs were orally administered according to the prescribed dosage regimen.

[Table 1]

**Table 1**

| OA/SA ratio | Number of cases | Number of incidence of cardiac events (incidence) | Corrected hazard ratio | P value |
|---|---|---|---|---|
| - 2.6 | 5158 | 113 (2.2%) | 1.00 | |
| 2.6 - 3.0 | 5107 | 160(3.1%) | 1.16 | 0.244 |
| 3.0 - | 5269 | 230 (4.4%) | 1.30 | 0.026 |

As is clear from Table 1, it was confirmed that the risk for occurrence of cardiovascular events was significantly increased at an OA/SA ratio in plasma of at least 3.
The average and standard deviation of the OA/SA ratio in plasma in the control group and the EPA group before the start of the trial and during the whole observation period are shown in Table 2.

[Table 2]

**Table 2**

| | | Before start of trial | | During whole observation period | |
|---|---|---|---|---|---|
| | | Average | S.D. | Average | S.D. |
| OA/SA ratio | Control group | 2.98 | 0.62 | 2.93 | 0.52 |
| | EPA group | 2.96 | 0.60 | 2.78 | 0.60 |

As is clear from Table 2, a significant decrease of the OA/SA ratio in plasma was confirmed after the administration of EPA-E. Therefore, the EPA-E administration reduces the incidence and/or rate of recurrence of cardiovascular events.

### (Example 2)

### (Plasma OA or Plasma SA, and Effect of EPA-E Administration)

### Trial procedure

This trial corresponds to a partial analysis of the results obtained in JELIS as in Example 1. More specifically, primary prevention cases combined with secondary prevention cases, only primary prevention cases, and only secondary prevention cases were respectively divided into four groups based on the plasma OA level or the plasma SA level at the start of administration so that the four groups were approximately equal in number. The groups were compared for the incidence of cardiovascular events for 5 years from the start of administration. The comparison results are shown in Tables 3 to 8.

Table 3 shows the incidence of cardiovascular events in the total of the primary prevention cases and the secondary prevention cases based on the plasma OA level at the start of administration.
[Table 3]

**Table 3**

| Oleic acid (mol%) | EPA-E group | | | Control group | | | Ratio of incidence reduction (%) |
|---|---|---|---|---|---|---|---|
| | Number of cardiovascular events / total number of cases | | Incidence (%) | Number of cardiovascular events / total number of cases | | Incidence (%) | |
| - 18.2 | 58 | 1762 | 3.3 | 56 | 1792 | 3.1 | -5.3 |
| >18.2-20.1 | 55 | 1788 | 3.1 | 69 | 1916 | 3.6 | 14.6 |
| >20.1-22.1 | 76 | 1733 | 4.4 | 79 | 1838 | 4.3 | -2.0 |
| 22.1 < | 77 | 1650 | 4.7 | 121 | 1808 | 6.7 | 30.3 |
| Total | 266 | 6933 | 3.8 | 325 | 7354 | 4.4 | 13.2 |

Table 4 shows the incidence of cardiovascular events in the total of the primary prevention cases and the secondary prevention cases based on the plasma SA level at the start of administration.
[Table 4]

**Table 4**

| Stearic acid (mol%) | EPA-E group | | | Control group | | | Ratio of incidence reduction (%) |
|---|---|---|---|---|---|---|---|
| | Number of cardiovascular events / total number of cases | | Incidence (%) | Number of cardiovascular events / total number of cases | | Incidence (%) | |
| - 6.46 | 85 | 1782 | 4.8 | 89 | 1833 | 4.9 | 1.8 |
| >6.46-6.89 | 81 | 1656 | 4.9 | 80 | 1863 | 4.3 | -13.9 |
| >6.89-7.35 | 56 | 1754 | 3.2 | 81 | 1862 | 4.4 | 26.6 |
| 7.35 < | 44 | 1742 | 2.5 | 75 | 1796 | 4.2 | 39.5 |
| Total | 266 | 6934 | 3.8 | 325 | 7354 | 4.4 | 13.2 |

Table 5 shows the incidence of cardiovascular events in the primary prevention cases based on the plasma OA level at the start of administration.
[Table 5]

**Table 5**

| Oleic acid (mol%) | EPA-E group | | | Control group | | | Ratio of incidence reduction (%) |
|---|---|---|---|---|---|---|---|
| | Number of cardiovascular events / total number of cases | | Incidence (%) | Number of cardiovascular events / total number of cases | | Incidence (%) | |
| - 18.1 | 27 | 1461 | 1.8 | 24 | 1457 | 1.6 | -12.2 |
| >18.1-19.9 | 27 | 1402 | 1.9 | 40 | 1535 | 2.6 | 26.1 |
| > 19.9-21.9 | 46 | 1422 | 3.2 | 41 | 1528 | 2.7 | -20.6 |
| 21.9 < | 40 | 1417 | 2.8 | 62 | 1529 | 4.1 | 30.4 |
| Total | 140 | 5702 | 2.5 | 167 | 6049 | 2.8 | 11.1 |

Table 6 shows the incidence of cardiovascular events in the primary prevention cases based on the plasma SA level at the start of administration.
[Table 6]

**Table 6**

| Stearic acid (mol%) | EPA-E group | | | Control group | | | Ratio of incidence reduction (%) |
|---|---|---|---|---|---|---|---|
| | Number of cardiovascular events / total number of cases | | Incidence (%) | Number of cardiovascular events / total number of cases | | Incidence (%) | |
| - 6.46 | 36 | 1483 | 2.4 | 47 | 1510 | 3.1 | 22.0 |
| >6.46-6.89 | 43 | 1373 | 3.1 | 41 | 1535 | 2.7 | -17.3 |
| >6.89-7.33 | 34 | 1404 | 2.4 | 45 | 1496 | 3.0 | 19.5 |
| 7.33 < | 27 | 1442 | 1.9 | 34 | 1508 | 2.3 | 17.0 |
| Total | 140 | 5702 | 2.5 | 167 | 6049 | 2.8 | 11.1 |

Table 7 shows the incidence of cardiovascular events in the secondary prevention cases based on the plasma OA level at the start of administration.
[Table 7]

**Table 7**

| Oleic acid (mol%) | EPA-E group | | | Control group | | | Ratio of incidence reduction (%) |
|---|---|---|---|---|---|---|---|
| | Number of cardiovascular events / total number of cases | | Incidence (%) | Number of cardiovascular events / total number of cases | | Incidence (%) | |
| - 18.2 | 34 | 303 | 11.2 | 37 | 320 | 11.6 | 3.0 |
| >18.2-20.1 | 28 | 322 | 8.7 | 28 | 333 | 8.4 | -3.4 |
| >20.1-22.1 | 37 | 322 | 11.5 | 41 | 328 | 12.5 | 8.1 |
| 22.1 < | 27 | 284 | 9.5 | 52 | 324 | 16.0 | 40.8 |
| Total | 126 | 1231 | 10.2 | 158 | 1305 | 12.1 | 15.5 |

Table 8 shows the incidence of cardiovascular events in the secondary prevention cases based on the plasma SA level at the start of administration. [Table 8]

**Table 8**

| Stearic acid (mol%) | EPA-E group | | | Control group | | | Ratio of incidence reduction (%) |
|---|---|---|---|---|---|---|---|
| | Number of cardiovascular events / total number of cases | | Incidence (%) | Number of cardiovascular events / total number of cases | | Incidence (%) | |
| - 6.46 | 49 | 299 | 16.4 | 42 | 323 | 13 | -26.0 |
| >6.46-6.90 | 41 | 289 | 14.2 | 40 | 338 | 11.8 | -19.9 |
| >6.90-7.38 | 22 | 317 | 6.9 | 37 | 327 | 11.3 | 38.7 |
| 7.38 < | 14 | 327 | 4.3 | 39 | 317 | 12.3 | 65.2 |
| Total | 126 | 1232 | 10.2 | 158 | 1305 | 12.1 | 15.5 |

As is clear from Tables 3 to 8, the EPA-E administration reduced incidence and/or rate of recurrence of cardiovascular events in patients with a high plasma OA level or a high plasma SA level (patients with an OA level exceeding 22.1 mol% or an SA level exceeding 7.38 mol%).

## Claims

1. A pharmaceutical composition for reducing a ratio of oleic acid to stearic acid in plasma of a patient, the pharmaceutical composition comprising ethyl icosapentate as its effective component.

2. The pharmaceutical composition according to claim 1, wherein the ratio of oleic acid to stearic acid in plasma of the patient is high.

3. The pharmaceutical composition according to claim 1 or 2 for reducing the risk of the patient at high risk for occurrence and/or recurrence of cardiovascular events.

4. The pharmaceutical composition according to any one of claims 1 to 3, wherein the patient suffers from hyperlipidemia and/or dyslipidemia.

5. The pharmaceutical composition according to any one of claims 1 to 4 for administering to the patient with a ratio of oleic acid to stearic acid in plasma of at least 3.

6. The pharmaceutical composition according to any one of claims 1 to 5, wherein administration of the composition to the patient with a ratio of oleic acid to stearic acid in plasma of at least 3 is started, continued until the ratio of oleic acid to stearic acid in plasma is reduced to less than 2.6 and further continued so that the ratio of oleic acid to stearic acid in plasma is kept at less than 2.6.

7. The pharmaceutical composition according to any one of claims 1 to 6 which is used in combination with a 3-hydroxy-3-methylglutaryl-coenzyme A reductase inhibitor.

8. A high-risk patient selecting method for selecting a patient at high risk for occurrence and/or recurrence of cardiovascular events, wherein a ratio of oleic acid to stearic acid in plasma is used as a marker.

9. A method for preventing occurrence and/or recurrence of cardiovascular events by keeping at less than 2.6 a ratio of oleic acid to stearic acid in plasma of a patient who has a high ratio of oleic acid to stearic acid in plasma.

10. The method according to 9 which comprises administering a pharmaceutical composition comprising ethyl icosapentate as its effective component.

11. A method for preventing occurrence and/or recurrence of cardiovascular events in humans, wherein ethyl icosapentate is administered to keep a ratio of oleic acid to stearic acid in human plasma at less than 2.6.
